(19) Europäisches Patentamt
European Patent Office
Office européen des brevets

(11) Veröffentlichungsnummer: **0 219 460 B1**

(12) # EUROPÄISCHE PATENTSCHRIFT

(45) Veröffentlichungstag der Patentschrift: **02.05.91**

(51) Int. Cl.5: **C07C 275/54**, C07C 217/84, C07C 265/12, A01N 47/34

(21) Anmeldenummer: **86810446.4**

(22) Anmeldetag: **08.10.86**

(54) **Benzoylphenylharnstoffe.**

(30) Priorität: **14.10.85 CH 4420/85**

(43) Veröffentlichungstag der Anmeldung:
**22.04.87 Patentblatt 87/17**

(45) Bekanntmachung des Hinweises auf die Patenterteilung:
**02.05.91 Patentblatt 91/18**

(84) Benannte Vertragsstaaten:
**AT BE CH DE ES FR IT LI NL**

(56) Entgegenhaltungen:
**DE-A- 2 726 684**

(73) Patentinhaber: **CIBA-GEIGY AG**
**Klybeckstrasse 141**
**CH-4002 Basel(CH)**

(72) Erfinder: **Drabek, Jozef, Dr.**
**Benkenstrasse 12**
**CH-4104 Oberwil(CH)**

EP 0 219 460 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue substituierte N-Benzoyl-N'-3,5-dichlor-4-hexafluorpropyloxyphenylharnstoffe, Verfahren und Zwischenprodukte zu ihrer Herstellung und ihre Verwendung in der Schädlingsbekämpfung.

Erfindungsgemäss werden Verbindungen der Formel I vorgeschlagen

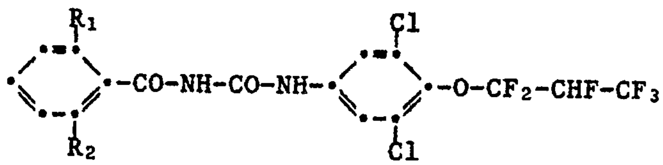 (I),

worin $R_1$ Wasserstoff oder Fluor und $R_2$ Fluor oder Chlor bedeuten, sowie deren Salze.

Hervorzuheben sind insbesondere diejenigen Verbindungen der Formel I, worin $R_1$ Fluor und $R_2$ Fluor oder Chlor bedeuten.

Weiterhin bevorzugte Verbindungen der Formel I sind dadurch gekennzeichnet, dass $R_1$ Wasserstoff und $R_2$ Fluor oder Chlor bedeuten.

Die Verbindungen der Formel I können analog an sich bekannter Verfahren hergestellt werden (vgl. z.B. die deutschen Offenlegungsschriften Nr. 2.123.236, 2.601.780 und 3.240.975).

So kann man z.B. eine Verbindung der Formel I erhalten, durch Umsetzung

a) der Verbindung der Formel II

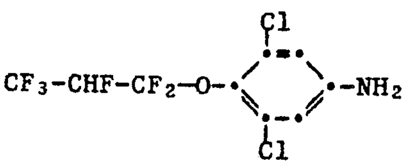 (II)

mit einer Verbindung der Formel III

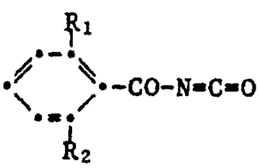 (III)

oder

b) der Verbindung der Formel IV

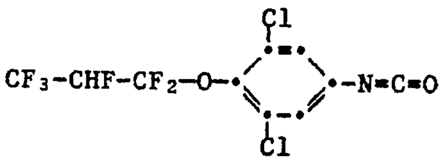 (IV)

mit einer Verbindung der Formel V

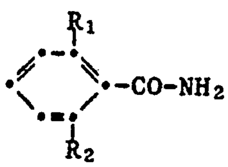 (V),

oder
c) der Verbindung der Formel II mit einer Verbindung der Formel VI

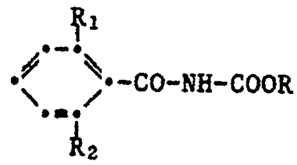

(VI).

In den obigen Formeln III, V und VI haben die Reste $R_1$ und $R_2$ die unter Formel I vorstehend angegebenen Bedeutungen, und R bedeutet einen $C_1$-$C_8$-Alkylrest, der gegebenenfalls mit Halogen, vorzugsweise Chlor, substituiert ist.

Die erwähnten Verfahren a), b) und c) können vorzugsweise unter normalem Druck und in Gegenwart eines organischen Lösungs- oder Verdünnungsmittels durchgeführt werden. Als Lösungs-oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis +200° C, vorzugsweise zwischen 0 und 100° C, z.B. bei Raumtemperatur, gegebenenfalls in Gegenwart einer organischen Base, z.B. Triäthylamin, durchgeführt. Die Durchführung von Verfahren b) erfolgt bei einer Temperatur von 0 bis 150° C vorzugsweise beim Siedepunkt des verwendeten Lösungsmittels, und gegebenenfalls in Gegenwart einer organischen Base, wie Pyridin, und/oder unter Zusatz eines Alkali-oder Erdalkalimetalls, vorzugsweise Natrium. Für das Verfahren c), d.h. für die Umsetzung der Urethane der Formel VI mit einem Anilin der Formel II, werden Temperaturen zwischen etwa 60° C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III und V sind bekannt und können analog bekannter Verfahren hergestellt werden. Bei dem Ausgangs-Anilin der Formel II handelt es sich um eine neuartige Verbindung, die ebenfalls einen Gegenstand der vorliegenden Erfindung bildet.

Die Verbindung der Formel II kann in an sich bekannter Weise dadurch hergestellt werden, dass man das entsprechend substituierte Nitrobenzol der Formel VII

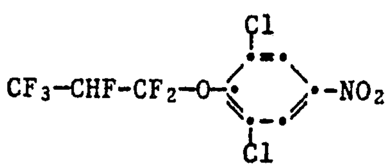

(VII)

in Analogie zu dem in J. Org. Chem. 29 (1964), 1, aufgezeigten Verfahren hydriert (vgl. auch die dort zitierte Literatur). Aber auch durch chemische Reduktion (z.B. mittel s Sn-(II)-Chlorid/HCl) der Nitroverbindung der Formel VII ist das Anilin der Formel II zugänglich (Vgl. Houben Weyl, "Methoden d. org. Chemie" 11/1, 422). Die Nitroverbindung der Formel VII selbst ist herstellbar durch Fluoralkylierung von 2,6-Dichlor-4-Nitrophenol. Eine weitere Herstellungsmöglichkeit für das Anilin der Formel II besteht darin, dass man das acylierte 3,5-Dichlor-4-hydroxyanilin in entsprechender Weise fluoralkyliert und dann die Acylgruppe entfernt, z.B. durch saure Hydrolyse, oder die Fluoralkylierung mit einem Salz des 3,5-Dichlor-4-hydroxyanilins, z.B. dem Chlorhydrat, durchführt.

Zu Benzoylisocyanaten gemäss Formel III kann man unter anderem wie folgt gelangen (vgl. J. Agr. Food Chem. 21, 348 und 993; 1973):

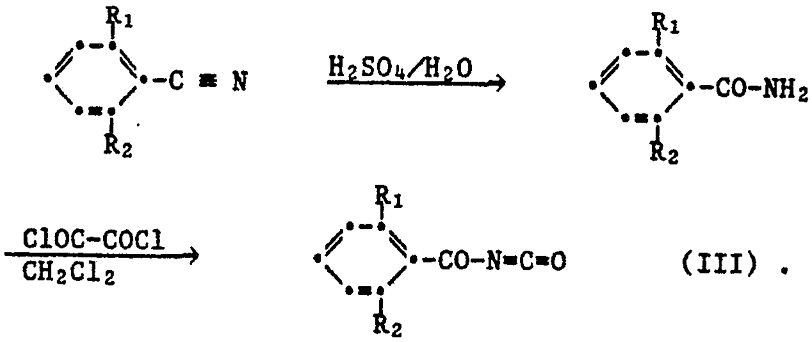


(III) .

Das an sich neuartige 4-(Hexafluorpropyloxy)-phenylisocyanat der Formel IV (Sdp. 95 °C/0,01 Torr.) lässt sich z.B. durch Phosgenierung des Anilins der Formel II nach allgemein üblichen Verfahren herstellen und bildet auch einen Gegenstand der Erfindung. Die weiterhin als Ausgangsstoffe zu verwendenden Benzamide der Formel V sind bekannt (vgl. z.B. Beilstein "Handbuch der organischen Chemie" Bd. 9, S. 336).

Urethane der Formel VI können in an sich bekannter Weise erhalten werden durch Umsetzung eines Benzoylisocyanats der Formel III mit einem entsprechenden Alkohol oder durch Umsetzung eines Benzamides der Formel V in Anwesenheit einer basischen Verbindung mit einem entsprechenden Ester der Chlorameisensäure Cl-COOR.

Zu den neuartigen Verbindungen der Formel I gehören erfindungsgemäss auch deren Salze, die sich nicht nur durch hohe insektizide Wirkung, sondern auch durch gute Löslichkeit in Lösungs-und Verdünnungsmitteln, insbesondere in organischen Lösungsmitteln, sowie durch verbesserte Formulierbarkeit auszeichnen.

Hervorzuheben sind die Metallsalze der erfindungsgemässen Verbindungen der Formel I, insbesondere ihre Alkali- und Erdalkalisalze, vorzugsweise die Natriumsalze und Kaliumsalze. Diese Salze werden in an sich bekannter Weise hergestellt, z.B. durch Umsetzen einer Verbindung der Formel I mit einem Metallalkanolat, wie Natriumäthylat oder Kaliummethylat. Bei einem vorgegebenen Salz können durch Umsalzen erwünschte Salze anderer Metalle erhalten werden.

Von besonderer Bedeutung sind die Salze der Verbindungen der Formel I mit organischen Basen, die im wesentlichen gekennzeichnet sind durch das Vorliegen eines quarternären Stickstoffatoms. Diese Salze entsprechen der Formel Ia

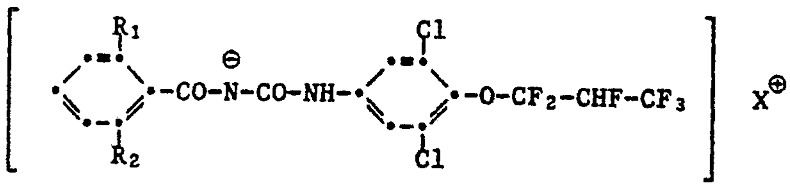

(Ia),

wobei $R_1$ und $R_2$ die vorstehend angegebenen Bedeutungen besitzen und $X^{\oplus}$ das Kation einer organischen Base bedeute. Vorzugsweise steht $X^{\oplus}$ für folgende organische Kationen:

$(CH_3)_4\ N^{\oplus}$, $(C_2H_5)_4\ N^{\oplus}$, $(n\text{-}C_3H_7)_4\ N^{\oplus}$, $(i\text{-}C_3H_7)_4\ N^{\oplus}$,

$(n\text{-}C_4H_9)_4\ N^{\oplus}$, 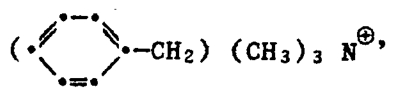

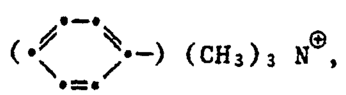 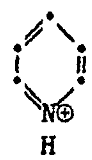 , $(C_4H_9)_3\ \overset{\oplus}{N}H$ ,

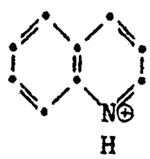 und $\left[ CH_3\text{-}(CH_2)_n\text{-} \right]_3 \overset{\oplus}{N}\text{—}CH_3$ ,

wobei n eine Zahl 8 bis 12 bedeutet. Unter den Salzen gemäss Formel Ia) sind auch Gemische dieser Salze mit unterschiedlichen Kationen zu verstehen. Die Salze der Formel Ia können in an sich bekannter Weise hergestellt werden durch Umsetzung einer Verbindung der Formel I mit entsprechenden Ammoniumhydroxyden der Formel $X^{\oplus}(OH)^{\ominus}$ , wobei $X^{\oplus}$ die vorstehend angegebene Bedeutung hat.

Es sind bereits N-Benzoyl-N'-2,5-dichlorphenyl-harnstoffe mit einem Halogenalkoxysubstituenten in 4-Stellung am Phenylring als Insektizide bekannt (vgl. US-Patentschrift Nr. 4.518.804 und deutsche Offenlegungsschrift Nr. 2848794). In der US-Patentschrift Nr. 4.162.330 werden insektizid wirksame N-Halogenbenzoyl-N'-(3,5-di-chlor-4-halogenalkenyloxy-phenyl)-harnstof e beschrieben. Insektizide N-Halogenbenzoyl-N'-(3,5-dichlor-4-halogenalkoxy-phenyl)-harnstoffe sind Gegenstand der US-Patentschrift Nr. 4.468.405. Verbindungen ähnlicher Struktur sind ferner aus der US-Patentschrift Nr. 4.529.819 und den britischen Patentanmeldungen Nr 2.106.499, 2.106.500, 2.106.501 und 2.113.679 bekannt. Gegenüber den in diesen Schrifttumstellen beschriebenen Verbindungen weisen die erfindungsgemässen neuartigen Benzoylphenylharnstoffe der Formel I als wesentliches unterscheidendes Strukturmerkmal eine in 4-Stellung am Phenylring befindliche 1,1,2,3,3,3-Hexafluorpropyloxy-Gruppe auf.

Ueberraschenderweise wurde gefunden, dass die vorliegenden Verbindungen der Formel I und ihre Salze bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekampfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte ovizide und vor allem larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Broybia rubrioculus, Panonychus citri, Eriophyes piri, Eriophyes ribis, Eriophyes vitis, Tarsonemus pallidus, Phyllocoptes vitis und Phyllocoptruta oleivora.

Die erfindungsgemässen Benzoylharnstoffe der Formel I weisen ferner Eigenschaften auf, die ihren

Einsatz zur Bekämpfung von Schnecken ermöglichen. Die repellierende und frasshemmende Wirkung dieser Verbindungen ist im Laborversuch häufig nur schwierig erkennbar. Im Freiland werden jedoch in Landwirtschaftlichen und gärtnerischen Pflanzenkulturen auch bei sehr niedrigen Aufwandmengen gute Wirkungen beobachtet. Insbesondere werden empfindliche Salat-, Gemüse- und Obstkulturen (wie Erdbeerkulturen) sowie die Kulturen von Zierpflanzen und Blumen gegen Schneckenfrass geschützt. Die Wirksamkeit der erfindungsgemässen Verbindungen der Formel I erstreckt sich auf alle Nackt- und Gehäuseschnekken, welche in der Mehrzahl als polyphage Schädlinge landwirtschaftlicher und gärtnerischer Kulturen auftreten. Es kann sowohl frasshemmende als auch abtötende Wirkung eintreten. Zu den landlebenden Schnecken gehören einige besonders wichtige Schädlinge wie z.B. die Nacktschnecken Arion rufus (Grosse Wegschnecke); Arion ater und andere Arionidae, Limax-Arten und die Ackerschnecken, u.a. Deroceras reticulatum und D. agreste aus der Familie Limacidae, sowie Arten aus der Familie Milacidae. Weiterhin schädigen auch die Gehäuseschnecken u.a. der Gattungen Bradybaena, Cepaea, Cochlodina, Discus, Euomphalia, Galba, Helicigona, Helix, Helicella, Helicodiscus, Lymnaea, Opeas, Vallonia und Zonitoides.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate,

Fettsulfate, sulfonierte Benzimid azolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykolräthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substtuenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

" Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag
München/Wien 1981.

Die pestiziden Zubereitungen enthalten in der Regel 0.1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25%, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 3,5-Dichlor-4-(1,1,2,3,3,3-hexafluorpropyloxy)-anilin:

Es werden 47 g 4-Acetylamino-3,5-dichlorphenol zusammen mit 154 g 90%iger Kaliumhydroxidlösung und 130 ml Dimethylformamid in einem Autoklaven verrührt. In den geschlossen en Autoklaven werden dann 75,8 g Hexafluorpropylen eingepresst. Das Gemisch wird während 20 Stunden bei 70°C unter dem sich im Autoklaven einstellenden Druck gerührt. Nach dem Abkühlen wird das Gemisch am Rotationsverdampfer eingeengt und der Rückstand in Methylenchlorid gelöst. Die erhaltene Lösung wird mit Wasser gewaschen, über $Na_2SO_4$ getrocknet und eingeengt. Das als Rückstand erhaltene Rohprodukt wird über eine Kieselgel-Säule (Länge 1 m, Durchmesser: 10 cm) mit einem Gemisch von Toluol und Aceton (11:1) chromatographiert. Man erhält das 4-Acetylamino-3,5-dichlor-1-(1,1,2,3,3,3-hexafluorpropyloxy)-benzol als hellgelbe Kristalle (Smp.: 113-115°C), wovon 26 g mit 110 ml Aethanol und 35,6 ml 37%iger Salzsäure während 10 Stunden am Rückfluss gehalten werden. Das Reaktionsgemisch wird dann eingeengt, mit Eis/Wasser verdünnt und schwach alkalisch gestellt. Das Produkt wird aus dem Gemisch mit Methylenchlorid extrahiert. Die organische Extrakt-Phase wird mit Wasser gewaschen, über $Na_2SO_4$

getrocknet und eingeengt. Der Rückstand wird durch Destillation gereinigt. Auf diese Weise erhält man die Titelverbindung der Formel

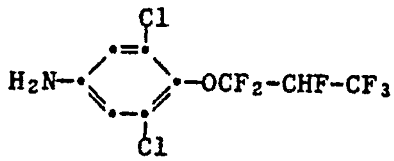

als farblose Flüssigkeit vom Siedepunkt 91-96 ° C/0.001 Torr.

b) Herstellung von N-(2,6-Difluorbenzoyl)-N'-[3,5-dichlor-4-(1,1,2,3,3,3-hexafluorpropyloxy)-phenyl]-harnstoff:

4,3 g 3,5-Dichlor-4-(1,1,2,3,3,3-hexafluorpropyloxy)-anilin werden unter Rühren in 50 ml trockenem Toluol gelöst und bei Raumtemperatur unter Feuchtigkeitsausschluss mit 2,41 g 2,6-Difluorbenzoylisocyanat, gelöst in 10 ml trockenem Toluol, versetzt. Der Ansatz wird während 12 Stunden bei Raumtemperatur weitergerührt. Danach werden am Rotationsverdampfer etwa 75 % des Lösungsmittels entfernt, der ausgefallene Rückstand wird abgenutscht, mit wenig kaltem Toluol und Hexan gewaschen und dann im Vakuum getrocknet.

Man erhält so die Titelverbindung der Formel

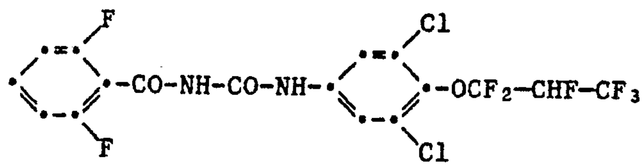

als farblose Kristalle mit einem Schmelzpunkt von 171-172 ° C (Verbindung Nr. 1).

In entsprechender Weise wie vorstehend beschrieben werden auch die folgenden Verbindungen der Formel I hergestellt:

| Verbindung Nr. | | Smp. |
|---|---|---|
| 2 | 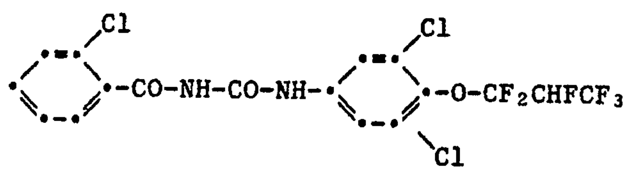 | 178-180°C |
| 3 | 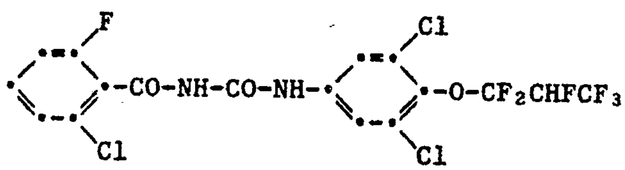 | 177-178°C |
| 4 | 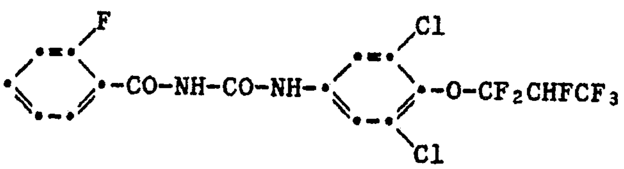 | 156-158°C |

c) Herstellung des Natriumsalzes der Verbindung Nr. 1:

9.58 g N-(2,6-Difluorbenzoyl)-N'-[3,5-dichlor-4-(1,1,2,3,3,3-hexafluorpropyloxy)-phenyl]-harnstoff werden in 20 ml absolutem Methanol suspendiert. Eine Lösung von 0,43 g Natrium in 30 ml absolutem Methanol

wird zu dieser Suspension unter Rühren zugetropft. Es entsteht eine klare Lösung; diese wird eingeengt und das zurückbleibende Produkt bei Raumtemperatur im Vakuum getrocknet. Man erhält so die Titelverbindung der Formel

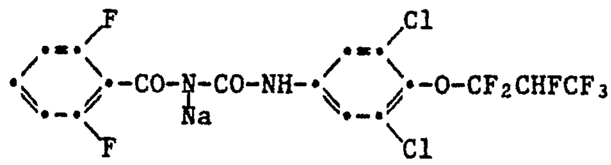

als farblose Kristalle.

Ferner ist wie vorstehend beschrieben auch das folgende Salz der Verbindung Nr. 2 der nachstehenden Formel herstellbar:

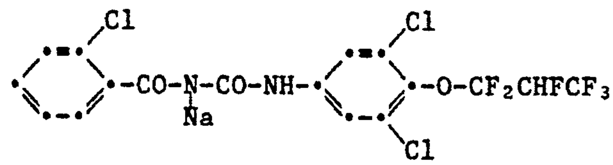

d) Herstellung des Tetrabutylammoniumsalzes der Verbindung Nr. 1:

Es werden 2,56 g N-(2,6-Difluorbenzoyl)-N'-[3,5-dichlor-4-(1,1,2,3,3,3-hexafluorpropyloxy)-phenyl]-harnstoff in 30 ml Methanol suspendiert. Unter Rühren gibt man zu dieser Suspension 5,2 g einer methanolischen Lösung enthaltend 1,3 g Tetra-n-butylammoniumhydroxyd. Es entsteht eine klare Lösung. Diese Lösung wird eingeengt und das als Rückstand zurückbleibende Rohprodukt in Hexan suspendiert, abgenutscht auf der Nutsche nochmals mit Hexan gewaschen und sodann getrocknet. Man erhält so die Titelverbindung der Formel

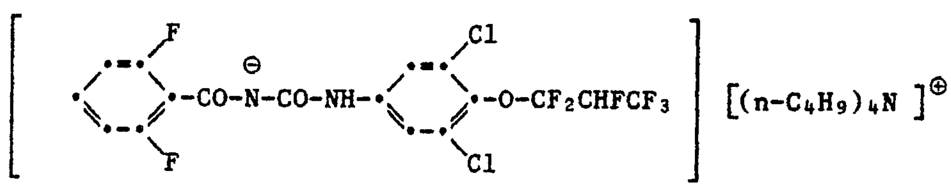

als farblose Kristalle.

Ferner können wie vorstehend beschrieben auch die folgenden Salze der Formel Ia hergestellt werden:

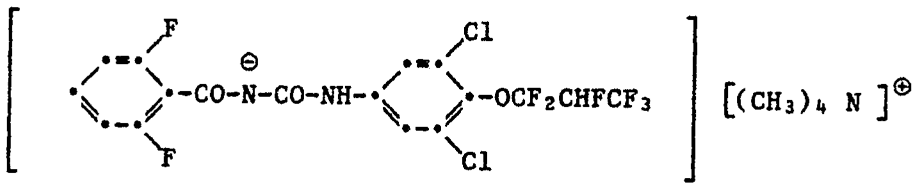

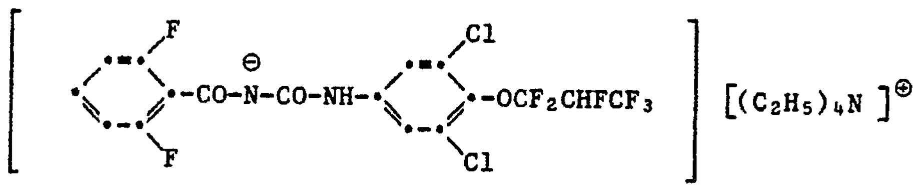

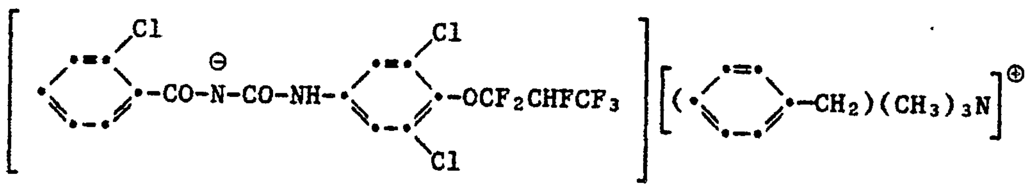

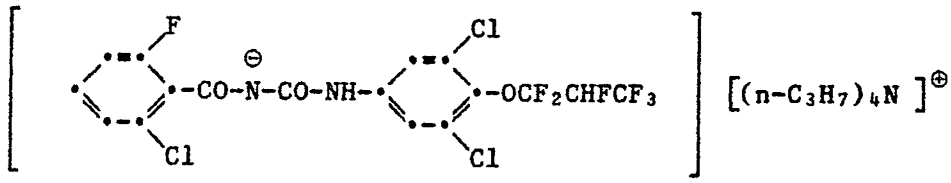

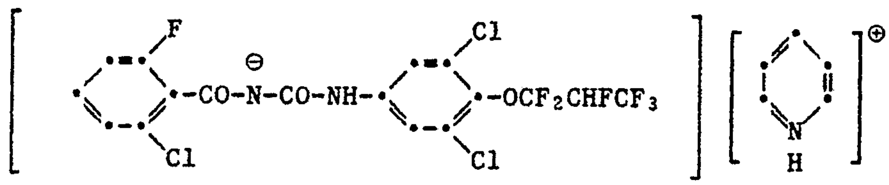

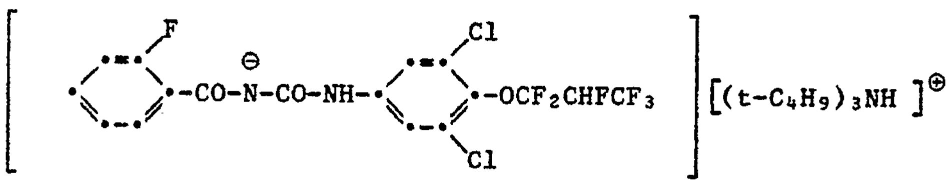

Beispiel 2: Formulierungen für Wirkstoffe der Formel I gemäss Beispiel 1 resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein SuspensionsKonzentrat, aus welchem durch Verdünnen mit Wasser Suspensionen jeder gewünschten Konzentration hergestellt werden können.

Beispiel 3: Wirkung gegen Musca domestica:

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 4: Wirkung gegen Lucilia sericata:

Zu 9 ml eines Zuchtmediums wird bei 50 °C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 5: Wirkung gegen Aëdes aegypti:

Auf die Oberfläche von 150 ml Was ser, das sich in einem Behälter befindet, wird so viel einer 0.1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 6: Insektizide Frassgift-Wirkung :

a) Ca. 25 cm hohe eingetopfte Baumwollpflanzen werden mit wässrigen Wirkstoffemulsionen besprüht,

die den Wirkstoff in Konzentrationen von 3, 12,5 und 50 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24° C und 60 % relativer Luftfeuchtigkeit durchgeführt. Nach 2 Tagen wird die %-Mortalität der Larven gegenüber unbehandelten Kontrollansätzen bestimmt.

Eine Wirkung von 80-100 % (Mortalität) zeigen die Verbindungen Nr. 1, 2, 3 und 4 gemäss Beispiel 1 bei 3 ppm gegen Spodoptera-Larven. Verbindung Nr. 1 zeigt bei 12,5 ppm und die Verbindungen Nr. 2 und 3 zeigen bei 50 ppm 80-100%ige Wirkung (Mortalität) gegen Heliothis-Larven.

b) Vergleichsversuch:

Der obige Versuch a) wird wiederholt unter Verwendung der erfindungsgemässen Verbindung Nr. 1 und zwei aus dem Stand der Technik vorbekannten Verbindungen. Abweichend von Versuch a) erfolgt die Bestimmung der Mortalität der Larven erst nach sieben Tagen. Die verwendeten Wirkstoffkonzentrationen, eingesetzten Verbindungen und erhaltenen Ergebnisse gehen aus der nachstehenden Tabelle hervor:

| verwendete Verbindungen | % Mortalität: Spodoptera littoralis 0,4 ppm | Spodoptera littoralis 0,8 ppm | Heliothis virescens 0,8 ppm | Heliothis virescens 1,5 ppm |
|---|---|---|---|---|
| 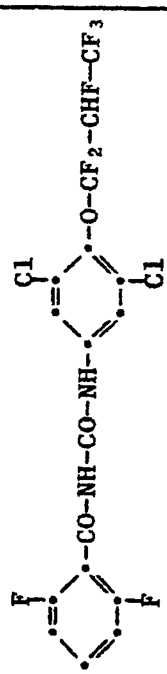 Verbindung Nr. 1 gemäss Beispiel 1 vorliegender Erfindung | 100 % | 100 % | 90 % | 100 % |
| 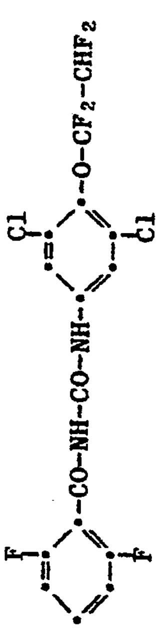 gemäss US-Patent Nr. 4.468.405 (Spalte 7/8, Verbindung Nr. 1) | 67 % | 90 % | 23 % | 70 % |
| 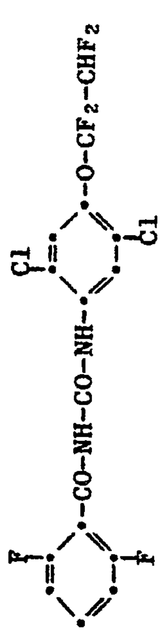 gemäss US-Patent 4.518.804 (Spalte 6, Verbindung "A") | 8 % | 10 % | 0 % | 0 % |

Beispiel 7: Wirkung gegen Epilachna varivestis:

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28 °C und 60 % relativer Luftfeuchtigkeit durchgeführt.

Nach 2 und 3 Tagen wird die % Mortalität bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss Beispiel 1 zeigen gute Wirkung im obigen Test.

Beispiel 8: Ovizide Wirkung auf Heliothis virescens:

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 800 ppm ergibt.

In diese wirkstoffhaltigen Emulsionen werden eintägige Eigelege von Heliothis auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Verbindungen der Formel I gemäss Beispiel 1 zeigen gute Wirkung in obigem Test.

Beispiel 9: Wirkung auf Laspeyresia pomonella (Eier):

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet und die %-Mortalit ät bestimmt.

Verbindungen der Formel I gemäss Beispiel 1 zeigen gute Wirkung in obigem Test.

Beispiel 10: Reproductions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 400 ppm des zu prüfenden Wirkstoffes eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird die %-Mortalität der Eier bestimmt, d.h. wieviel Larven sich aus den deponierten Eiern entwickelt haben.

Zur Ermittlung der Dauer des die Reproduktion beeinflussenden Effektes der zu prüfenden Wirkstoffe wird die Eiablage der Käfer noch weiter, d.h. während eines Zeitraums von etwa 4 Wochen überprüft. Die Bonitierung erfolgt anhand der Verminderung der Anzahl abgelegter Eier und daraus geschlüpfter Larven im Vergleich zu unbehandelten Kontrollen.

Verbindungen der Formel I gemäss Beispiel 1 zeigen gute Wirkung im obigen Test.

Beispiel 11: Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 1 zeigen gute Wirkung in obigen Test.

Beispiel 12: Insektizide Frassgift-Wirkung gegen Plutella xylostella

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 0,8 ppm enthalten und auf den Pflanzen antrocknen.

Nach zwei Tagen werden die behandelten Chinakohlpflanzen mit je 10 Plutella xylostella-Larven im zweiten larvalen Stadium besiedelt. Der Versuch wird bei 24°C und 60 % relativer Luftfeuchtigkeit im Dämmerlicht durchgeführt. Die Bonitur erfolgt nach 2 und 5 Tagen, es wird die %-Mortalität der Larven bestimmt.

Eine Wirkung von 100 % (Mortalität) zeigt die Verbindung Nr. 1 gemäss Beispiel 1 in diesem Test.

Beispiel 13: Frasshemmende Wirkung gegen Schnecken

Unter kontrollierten Versuchsbedingungen werden je 5 Nacktschnecken (Arion rufus) in einem Käfig mit 4 frischen Salatblättern über 17 Stunden belassen. Es handelt sich hierbei um Zwangsversuche, bei denen entweder nur durch eine Spritzapplikation behandelte oder unbehandelte Blätter angeboten werden. Die Wirkstoffkonzentration in der zur Applikation verwendeten wässrigen Zubereitung beträgt 0,5 Gew.-%. Das Ausmass des Frasses wird anhand von Gewichtsdifferenzen, Fotokopien des Frassbildes und nach augenscheinlichen Beurteilungskriterien gegenüber der unbehandelten Kontrolle ermittelt.

Daneben wird noch die allfällige Mortalität der Testtiere bestimmt.

Verbindungen der Formel I gemäss Beispiel 1 zeigen g ute Wirkung in obigem Test.

## Ansprüche

**1.** Verbindung der Formel I

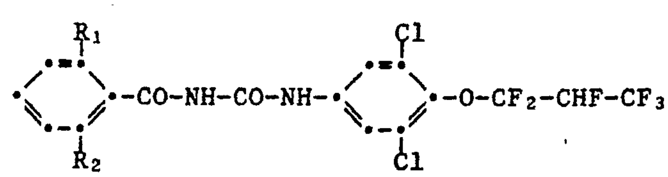

(I),

worin $R_1$ Wasserstoff oder Fluor und $R_2$ Fluor oder Chlor bedeuten, sowie deren Salze.

**2.** Verbindung der Formel I gemäss Anspruch 1, dadurch gekennzeichnet, dass $R_1$ Fluor und $R_2$ Fluor oder Chlor bedeuten.

**3.** Verbindung der Formel I gemäss Anspruch 1, dadurch gekennzeichnet, dass $R_1$ Wasserstoff und $R_2$ Fluor oder Chlor bedeuten.

**4.** Verbindung gemäss Anspruch 2 der Formel

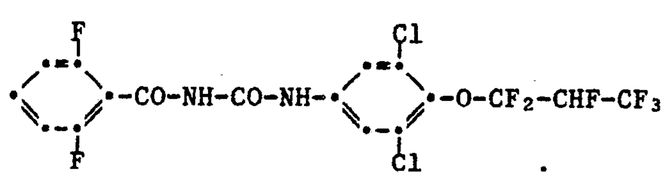

.

**5.** Verbindung gemäss Anspruch 3 der Formel

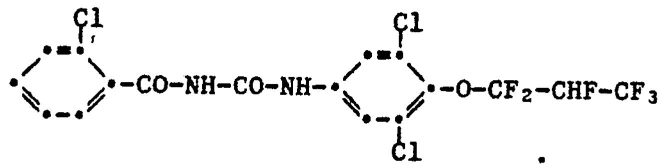
.

6. Verbindung gemäss Anspruch 3 der Formel

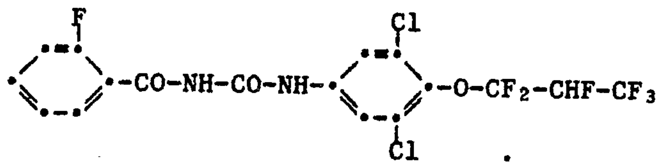
.

7. Verbindung gemäss Anspruch 2 der Formel

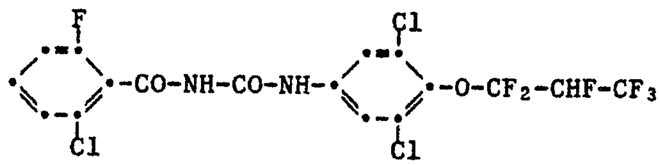
.

8. Metallsalz einer Verbindung der Formel I gemäss einem der Ansprüche 1 bis 7.

9. Salz einer Verbindung der Formel I gemäss einem der Ansprüche 1 bis 7 mit einer organischen Base.

10. Salz gemäss Anspruch 9, gekennzeichnet durch das Vorliegen eines quarternären Stickstoffatoms.

11. Verfahren zur Herstellung einer Verbindung gemäss einem der Ansprüche 1 bis 10 oder eines ihrer Salze, dadurch gekennzeichnet, dass man
a) die Verbindung der Formel II

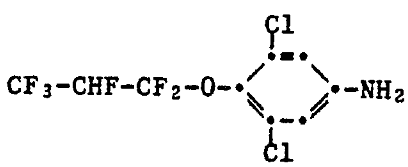
(II)

mit einer Verbindung der Formel III

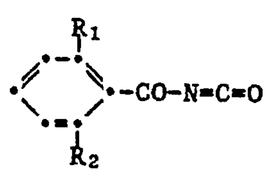
(III)

oder
b) die Verbindung der Formel IV

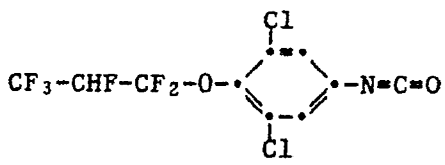 (IV)

mit einer Verbindung der Formel V

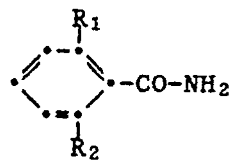 (V),

oder

c) die Verbindung der Formel II mit einer Verbindung der Formel VI

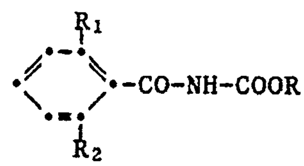 (VI)

umsetzt, wobei in den Formeln III, V und VI die Reste $R_1$ und $R_2$ die in den Ansprüchen 1 bis 3 angegebenen Bedeutungen haben und R für einen $C_1$-$C_8$-Alkylrest, der gegebenenfalls mit Halogen substsituiert ist, steht, und dass man die erhaltene Verbindung der Formel I gewünschtenfalls in eines ihrer Salze umwandelt.

**12.** Verbindung der Formel II

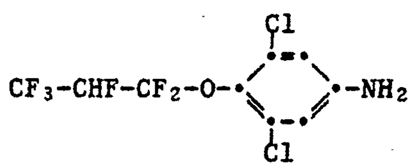 (II) .

**13.** Verbindung der Formel IV

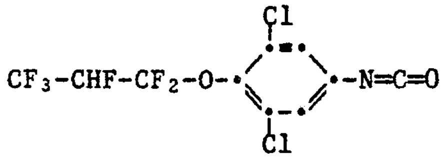 (IV) .

**14.** Schädlingsbekämpfungsmittel, welches als aktive Komponente eine Verbindung gemäss einem der Ansprüche 1 bis 7 oder eines ihrer Salze zusammen mit geeigneten Trägern und/oder anderen Zuschlagstoffen enthält

**15.** Verwendung einer Verbindung gemäss einem der Ansprüche 1 bis 7 oder eines ihrer Salze zur Bekämpfung von Insekten, Vertretern der Ordnung Akarina und Schnecken.

**16.** Verwendung gemäss Anspruch 15 zur Bekämpfung larvaler Stadien pflanzenschädigender Insekten.

**17.** Verwendung gemäss Anspruch 15 zur Bekämpfung von Insekten-Eiern.

**18.** Verfahren zur Bekämpfung von Insekten, Vertretern der Ordnung Akarina und Schnecken, dadurch gekennzeichnet, dass man diese Schädlinge bzw. deren verschiedene Entwicklungsstadien oder deren Aufenthaltsort mit einer pestizid wirksamen Menge einer Verbindung der Formel I gemäss einem der Ansprüche 1 bis 7 oder eines ihrer Salze oder mit einem Mittel enthaltend neben Zusatz-und Trägerstoffen eine pestizid wirksame Menge dieser Verbindung, in Kontakt bringt oder behandelt.

## Claims

**1.** A compound of formula I

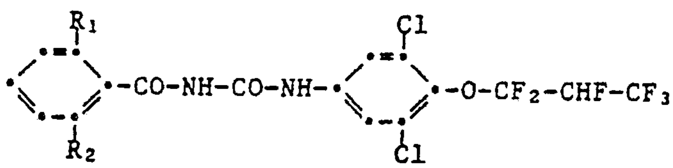

(I),

wherein $R_1$ is hydrogen or fluorine and $R_2$ is fluorine or chlorine, or a salt thereof.

**2.** A compound of formula I according to claim 1, wherein $R_1$ is fluorine and $R_2$ is fluorine or chlorine.

**3.** A compound of formula I according to claim 1, wherein $R_1$ is hydrogen and $R_2$ is fluorine or chlorine.

**4.** The compound according to claim 2 of the formula

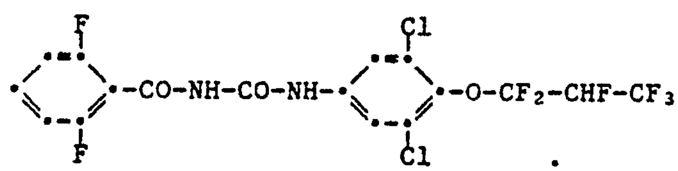

.

**5.** The compound according to claim 3 of the formula

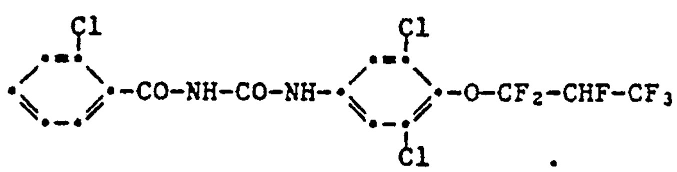

.

**6.** The compound according to claim 3 of the formula

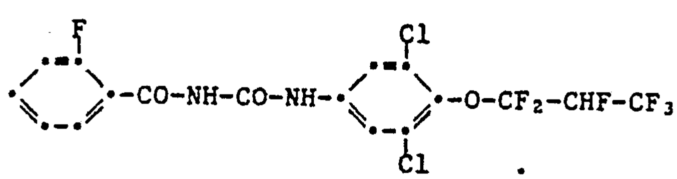

.

7. The compound according to claim 2 of the formula

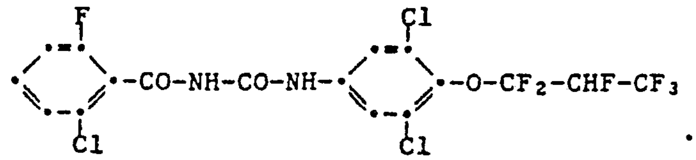

8. A metal salt of a compound of formula I according to any one of claims 1 to 7.

9. A salt of a compound of formula I according to any one of claims 1 to 7 with an organic base.

10. A salt according to claim 9, wherein a quaternary nitrogen atom is present.

11. A process for the preparation of a compound according to any one of claims 1 to 10, or of a salt thereof, which process comprises reacting

a) the compound of formula II

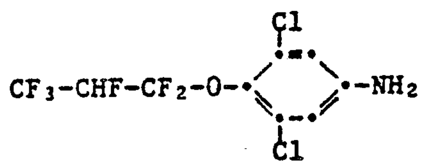

(II)

with a compound of formula III

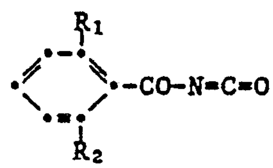

(III)

or

b) the compound of formula IV

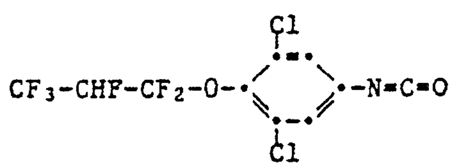

(IV)

with a compound of formula V

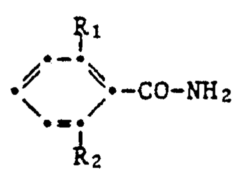

(V),

or

c) the compound of formula II with a compound of formula VI

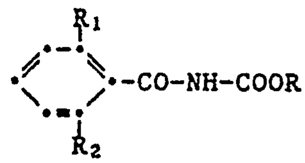 (VI)

in which in formulae III, V and VI the radicals $R_1$ and $R_2$ are as defined in claims 1 to 3 and R is a $C_1$-$C_8$ alkyl radical which is unsubstituted or substituted by halogen, and, if desired, converting the resultant compound of formula I into a salt thereof.

**12.** The compound of formula II

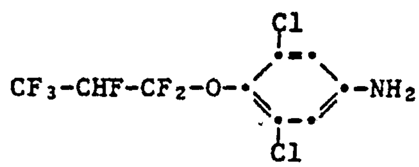 (II) .

**13.** The compound of formula IV

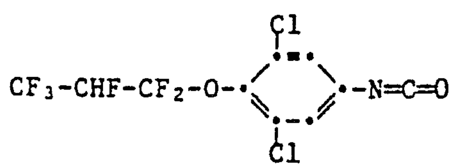 (IV) .

**14.** A pesticidal composition which contains as active ingredient a compound according to any one of claims 1 to 7, or a salt thereof, together with suitable carriers and/or other adjuvants.

**15.** Use of a compound according to any one of claims 1 to 7, or of a salt thereof, for controlling insects, representatives of the order Acarina and slugs and snails.

**16.** Use according to claim 15 for controlling larval stages of plant-destructive insects.

**17.** Use according to claim 15 for controlling insect eggs.

**18.** A method of controlling insects, representatives of the order Acarina and slugs and snails, which method comprises bringing into contact or treating said pests, the various development stages or the habitat thereof with a pesticidally effective amount of a compound of formula I according to any one of claims 1 to 7, or of a salt thereof, or with a composition which contains a pesticidally effective amount of such a compound, together with adjuvants and carriers.

## Revendications

**1.** Composé de formule I

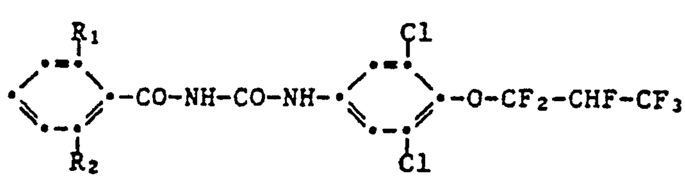 (I),

où $R_1$ représente l'hydrogène ou le fluor et $R_2$ représente le fluor ou le chlore ainsi que ses sels.

2. Composé de formule I selon la revendication 1, caractérisé en ce que $R_1$ représente le fluor et $R_2$ représente le fluor ou le chlore.

3. Composé de formule I selon la revendication 1, caractérisé en ce que $R_1$ représente l'hydrogène et $R_2$ représente le fluor ou le chlore.

4. Composé selon la revendication 2 de formule

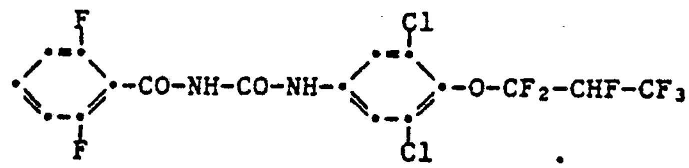

5. Composé selon la revendication 3 de formule

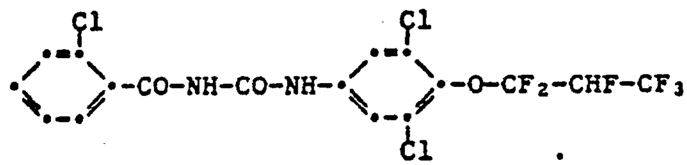

6. Composé selon la revendication 3 de formule

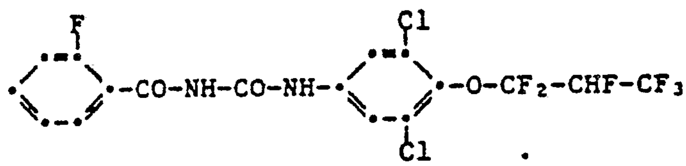

7. Composé selon la revendication 2 de formule

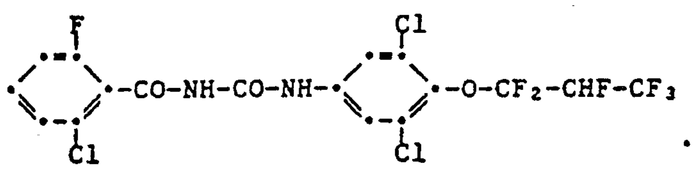

8. Sel métallique d'un composé de formule I selon l'une des revendications 1 à 7.

9. Sel d'un composé de formule I selon l'une des revendications 1 à 7 avec une base organique.

10. Sel selon la revendication 9, caractérisé par la présence d'un atome d'azote quaternaire.

11. Procédé pour la préparation d'un composé selon l'une des revendications 1 à 10 ou de l'un de ses sels, caractérisé en ce que l'on fait réagir
a) le composé de formule II

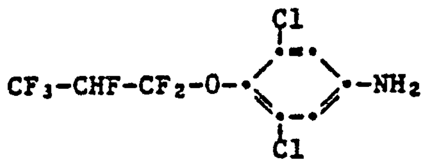

(II)

avec un composé de formule III

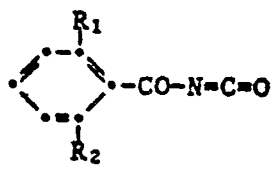

(III)

ou

b) le composé de formule IV

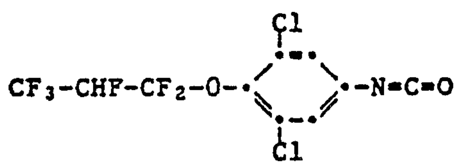

(IV)

avec un composé de formule V

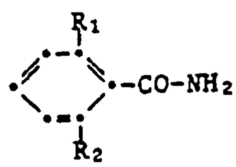

(V),

ou

c) le composé de formule II avec un composé de formule VI

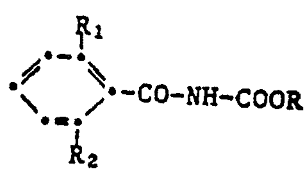

(VI)

les restes $R_1$ et $R_2$ dans les formules III, V et VI ayant les significations indiquées dans les revendications 1 à 3 et R représentant un reste alkyle en $C_1$-$C_8$ éventuellement substitué par un halogène et, si désiré, en ce que l'on transforme le composé de formule I obtenu en l'un de ses sels.

**12.** Composé de formule II

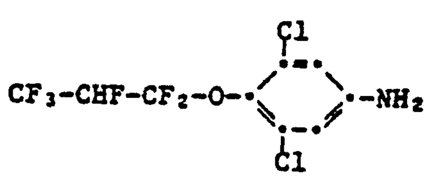

(II).

**13.** Composé de formule IV

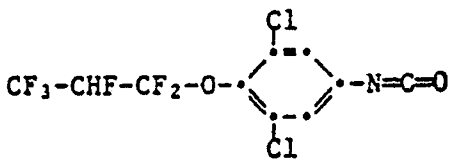

(IV).

**14.** Agent de lutte contre les parasites contenant comme composant actif un composé selon l'une des revendications 1 à 7 ou l'un de ses sels, associé à des supports appropriés et/ou d'autres additifs.

**15.** Utilisation d'un composé selon l'une des revendications 1 à 7 ou de l'un de ses sels pour la lutte contre les insectes, les représentants de l'ordre des acariens et les limaces.

**16.** Utilisation selon la revendication 15 pour la lutte contre les stades larvaires des insectes nuisibles aux plantes.

**17.** Utilisation selon la revendication 15 pour la lutte contre les oeufs d'insectes.

**18.** Procédé pour la lutte contre les insectes, les représentants de l'ordre des acariens et les limaces, caractérisé en ce que l'on amène en contact ou en ce que l'on traite ces parasites ou leurs différents stades de développement ou les endroits où ils séjournent avec une quantité efficace comme pesticide d'un composé de formule I selon l'une des revendications 1 à 7 ou de l'un de ses sels ou avec un produit contenant, à côté d'additifs et de supports, une quantité efficace comme pesticide de ce composé.